# EUROPEAN PATENT SPECIFICATION

(11) **EP 4 398 850 B1**
(45) Date of publication and mention of the grant of the patent: **25.11.2026**
(21) Application number: 22783118.7
(22) Date of filing: 07.09.2022
(51) Int. Cl.: A61F 5/451, A61F 5/455

(54) **FLUID COLLECTION ASSEMBLIES INCLUDING A FIRST POROUS MATERIAL EXHIBITING AT LEAST ONE OF A FLUID PERMEABILITY OR COMPRESSIBILITY THAT IS DIFFERENT THAN A SECOND POROUS MATERIAL**
FLÜSSIGKEITSSAMMELANORDNUNGEN MIT EINEM ERSTEN PORÖSEN MATERIAL MIT MINDESTENS EINER VON FLÜSSIGKEITSPERMEABILITÄT ODER KOMPRIMIERBARKEIT, DIE VERSCHIEDEN VON EINEM ZWEITEN PORÖSEN MATERIAL IST
ENSEMBLES DE COLLECTE DE FLUIDE COMPRENANT UN PREMIER MATÉRIAU POREUX PRÉSENTANT UNE PERMÉABILITÉ ET/OU UNE COMPRESSIBILITÉ DE FLUIDE DIFFÉRENTE D'UN SECOND MATÉRIAU POREUX

(30) Priority: 08.09.2021 US 202163241564 P
(43) Date of publication of application: 17.07.2024
(73) Proprietor: Purewick Corporation, El Cajon, California 92020 (US)
(72) Inventor: ROBICHAUD, Jonathan, Decatur, Georgia 30032 (US); ROTHBERG, Matthew Jordan, Atlanta, Georgia 30306 (US)
(74) Representative: Hoffmann Eitle
(86) International application number: PCT/US2022/042725
(87) International publication number: WO 2023/038950

(56) References cited:
- WO-A1-2020/256865
- WO-A1-2021/016026
- US-A1- 2021 228 795

## Description

### CROSS-REFERENCE TO RELATED APPLICATION(S)

This application claims priority to U.S. Provisional Patent Application No. 63/241,564 filed on September 8, 2021.

### BACKGROUND

A person or animal may have limited or impaired mobility so typical urination processes are challenging or impossible. For example, a person may experience or have a disability that impairs mobility. A person may have restricted travel conditions such as those experienced by pilots, drivers, and workers in hazardous areas. Additionally, sometimes bodily fluids collection is needed for monitoring purposes or clinical testing.

Urinary catheters, such as a Foley catheter, can address some of these circumstances, such as incontinence. Unfortunately, urinary catheters can be uncomfortable, painful, and can lead to complications, such as infections. Additionally, bed pans, which are receptacles used for the toileting of bedridden individuals are sometimes used. However, bedpans can be prone to discomfort, spills, and other hygiene issues.

### SUMMARY

The invention is defined in claim 1 below, in which the features of the precharacterizing part are disclosed in combination in Applicant's WO 2021/016026 A1. The dependent claims are directed to optional features and/or preferred embodiments of the invention. Embodiments are directed to fluid collection assemblies including a first porous material exhibiting at least one of a fluid permeability or compressibility that is different than a second porous material, and to fluid collection assemblies including the same. In an embodiment, a fluid collection assembly is disclosed. The fluid collection assembly includes a fluid impermeable layer including a proximal end region and a distal end region. The fluid impermeable layer at least defines at least one opening, a chamber, and a fluid outlet. The fluid collection assembly also includes a porous medium disposed in the chamber. The porous medium includes a proximal zone extending from or near the proximal end region of the fluid impermeable layer, a distal zone extending from or near the distal end region of the fluid impermeable layer to the proximal zone, a first porous material exhibiting a first fluid permeability and a first compressibility, and a second porous material exhibiting a second fluid permeability and a second compressibility. At least one of the first fluid permeability is greater than the second fluid permeability or the first compressibility is less than the second compressibility. The first porous material and the second porous material are disposed in the porous medium such that at least one of the proximal zone exhibits a fluid permeability that is greater than the fluid permeability of the distal zone or the proximal zone exhibits a compressibility that is less than a compressibility of the distal zone.

In an embodiment, a fluid collection system is disclosed. The fluid collection system includes a fluid collection assembly. The fluid collection assembly includes a fluid impermeable layer including a proximal end region and a distal end region. The fluid impermeable layer at least defines at least one opening, a chamber, and a fluid outlet. The fluid collection assembly also includes a porous medium disposed in the chamber. The porous medium includes a proximal zone extending from or near the proximal end region of the fluid impermeable layer, a distal zone extending from or near the distal end region of the fluid impermeable layer to the proximal zone, a first porous material exhibiting a first fluid permeability and a first compressibility, and a second porous material exhibiting a second fluid permeability and a second compressibility. At least one of the first fluid permeability is greater than the second fluid permeability or the first compressibility is less than the second compressibility. The first porous material and the second porous material are disposed in the porous medium such that at least one of the proximal zone exhibits a fluid permeability that is greater than the fluid permeability of the distal zone or the proximal zone exhibits a compressibility that is less than a compressibility of the distal zone. The fluid collection system also includes a fluid storage container and a vacuum source. The chamber of the fluid collection assembly, the fluid storage container, and the vacuum source are in fluid communication with each that, when one or more bodily fluids are present in the chamber, a vacuum provided from the vacuum source to the chamber of the fluid collection assembly removes the one or more bodily fluids from the chamber and deposits the bodily fluids in the fluid storage container.

Features from any of the disclosed embodiments may be used in combination with one another, without limitation. In addition, other features and advantages of the present disclosure will become apparent to those of ordinary skill in the art through consideration of the following detailed description and the accompanying drawings.

### BRIEF DESCRIPTION OF THE DRAWINGS

The drawings illustrate several embodiments of the present disclosure, wherein identical reference numerals refer to identical or similar elements or features in different views or embodiments shown in the drawings.
**FIG. 1A** is a top plan view of a fluid collection assembly.
**FIG. 1B** is a cross-sectional schematic of the fluid collection assembly taken along plane 1B-1B.
**FIGS. 2** and **3** are cross-sectional schematics for fluid collection assemblies that exhibit the same top plan view as the fluid collection assembly illustrated in **FIG. 1A****.**
**FIG. 4A** is a top plan view of a fluid collection assembly,.
**FIG. 4B** is a cross-sectional schematic of the fluid collection assembly taken along plane 4B-4B.
**FIG. 4C** is a cross-sectional schematic of a fluid collection assembly.
**FIG. 5** is a cross-sectional schematic of a fluid collection assembly.
**FIG. 6A** is a top plan view of a fluid collection assembly.
**FIG. 6B** is a cross-sectional schematic of the fluid collection assembly.
**FIG. 7** is a cross-sectional schematic of a fluid collection assembly.
**FIG. 8A** is a top plan view of a fluid collection assembly including a porous medium that has a third porous material in addition to the first porous material and the second porous material.
**FIG. 8B** is a cross-sectional schematic of the fluid collection assembly taken along plane 8B-8B.
**FIG. 9** is a cross-sectional schematic of a fluid collection assembly including a porous medium that has a third porous material in addition to the first porous material and the second porous material.
**FIG. 10** is a block diagram of a fluid collection system for fluid collection.

### DETAILED DESCRIPTION

Embodiments are directed to fluid collection assemblies including a first porous material exhibiting at least one of a fluid permeability or compressibility that is different than a second porous material, fluid collection assemblies including the same, and methods of using the same. An example fluid collection assembly includes a fluid impermeable layer *(e.g.,* a fluid impermeable barrier) at least defining at least one opening, a chamber, and a fluid outlet. The fluid collection assembly also includes a porous medium disposed in the chamber and extending across the opening. The porous medium includes a first porous material and a second porous material. The first porous material exhibits a first fluid permeability and a first compressibility. The second porous material exhibits a second fluid permeability and a second compressibility. In an embodiment, the first fluid permeability of the first porous material is greater than the second fluid permeability of the second porous material which may allow the porous medium to at least one receive one or more bodily fluids (e.g., urine) quicker or provide a more targeted vacuum (*e.g.*, suction). In an embodiment, the first compressibility of the first porous material is less than the second compressibility of the second porous material which may make the fluid collection assembly more comfortable to use (*e.g.*, prevent chaffing or other contact that may be deemed uncomfortable) while allowing unobstructed fluid flow.

During use, the fluid collection assemblies disclosed herein are positioned adjacent to a vaginal region of an individual. For example, the fluid collection assemblies disclosed herein may be positioned such that the portion of the porous medium extending across the opening is adjacent to or abuts the urethral opening of the individual. After positioning the fluid collection assembly, the individual may discharge one or more bodily fluids (*e.g.,* urine, blood, sweat, etc.). The bodily fluids may be received into the porous medium and the chamber. The bodily fluids then flow through the porous medium to a conduit that is in fluid communication with the chamber. The bodily fluids may then be removed from the chamber via the conduit thereby maintaining the individual dry. In some embodiments, a vacuum may be provided to the chamber via the conduit. The vacuum may help pull the bodily fluids through the porous medium, towards the conduit, and into the conduit.

As previously discussed, the porous medium is positioned adjacent to the vaginal region of the individual and, as such, it is desirably to form the porous medium from a comfortable porous material since the vaginal region of the individual is sensitive. Generally, comfortable porous materials are smooth to prevent chaffing and a compressible material to allow the porous medium to conform to and more uniformly apply pressure to the vaginal region. Also, the porous medium is configured to receive one or more bodily fluids discharged from the individual. When the individual urinates, a relatively large volume of bodily fluids may be discharged from the individual over a short period of time and the spray of the bodily fluids may be concentrated in a small portion of the porous medium. As such, it is desirably to form the porous medium from a material exhibiting a relatively high fluid permeability to allow the bodily fluids to be quickly received into the porous medium to prevent bodily fluids from leaking.

Generally, smooth materials exhibit relatively low fluid permeability since increasing the fluid permeability may increase the surface roughness of the porous material. For example, materials that are soft tend to have small pores, which limits the quantity of bodily fluids that may be received into the soft materials at any given time. Also, generally, compressible materials are likely to be compressed which may obstruct or otherwise close the passageways defined by the compressible material which may inhibit fluid flow therethrough. Materials that exhibit relatively high fluid permeability tend to define large pores which increase the surface roughness of such materials and are not compressible to prevent collapse of passageways defined thereby.

Conventional fluid collection assemblies solve these issues by forming a porous medium that includes an outer layer and an inner layer. To make the conventional fluid collection assemblies more comfortable to use, the outer layer is formed from a relatively compressible and/or smooth material compared to the inner layer. To improve fluid flow through the conventional fluid collection assemblies, the inner layer is formed from a material exhibiting a relatively high fluid permeability compared to the outer layer. The thickness of the inner and outer layers remain relatively constant along an entire length of the porous medium of the conventional fluid collection assemblies. During use, at least initially, the volume of the bodily fluids discharged is concentrated on a relatively small portion of the outer layer. However, the outer layer may not exhibit a fluid permeability sufficient to receive the bodily fluids discharged by the individual since the outer layer was selected based on compressibility and/or smoothness of the material instead of fluid permeability. As such, especially during large discharges of urine, the outer layer of conventional fluid collection assemblies may be unable to receive all of the discharged bodily fluids thereby causing the bodily fluids to leak. Additionally, in such conventional fluid collection assemblies, the vacuum tends to be introduced at a location of the porous medium that is spaced from the urethral opening of the individual. The uniform thickness of the outer and inner layers of porous medium of the conventional fluid collection assemblies means that the air flow caused by the vacuum is not directed to the portions of the porous medium adjacent to the urethral opening (*i.e.,* the portions of the porous medium that need the vacuum the most). Instead, the vacuum may dissipate (*e.g.,* leak from the porous medium) before reaching the portion of the porous medium that is adjacent to the urethral opening. Further, the thickness of the outer layer may be minimal which decreases any benefit caused by the compressibility of the outer layer.

The fluid collection assemblies disclosed herein use the different fluid permeabilities and/or compressibilities of the first and second porous materials to solve at least some of these issues of the conventional fluid collection assemblies. For example, the different fluid permeabilities and/or compressibilities of the first and second porous materials are configured to improve the rate at which bodily fluids are received into the porous material, direct the vacuum to selected portions of the porous material, or prevent collapse of the passageways through which the bodily fluids may flow while making the fluid collection assemblies as comfortable as possible. In an embodiment, the different fluid permeabilities and/or compressibilities of the first and second porous materials may be configured to form different zones in the porous medium, wherein different zones exhibiting different fluid permeabilities and/or compressibilities. The different zones may be used to improve fluid flow through the porous medium and/or make the porous medium more comfortable. In an example, the porous material may include a proximal zone and a distal zone. The proximal zone may include portions of the porous material that are likely to contact or otherwise be positioned proximate to the urethral opening during use while the distal zone is unlikely to contact the urethral opening. As such, the proximal zone may exhibit a fluid permeability that is greater than the fluid permeability of the distal zone and/or the proximal zone may exhibit a compressibility that is less than the distal zone which may improve the fluid flow through the porous medium at or near the urethral opening (*e.g.*, where the fluid flow through the porous medium is most important) and make the porous material more comfortable against portions of the vaginal region that are spaced from the urethral opening. The first and second porous materials may be disposed within the porous medium such that the porous medium includes the proximal and distal zones.

It is noted that, in some embodiments, fluid permeability may refer to the permeability of a vacuum through the porous medium in addition to the permeability of the bodily fluids through the porous medium. It is also noted that a material exhibiting a relatively high fluid permeability does not necessarily exhibit a relatively low compressibility. For example, some materials exhibiting high fluid permeability may exhibit a relatively high compressibility and some materials exhibiting low fluid permeability may exhibit a relatively low compressibility.

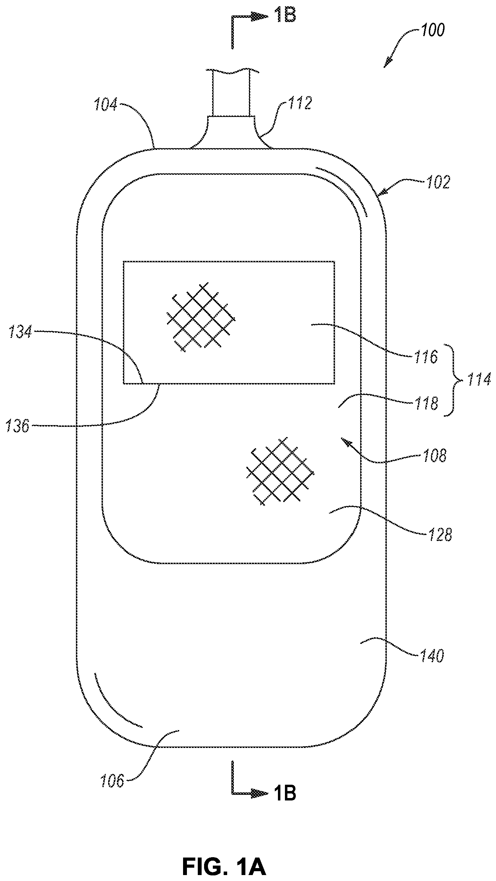

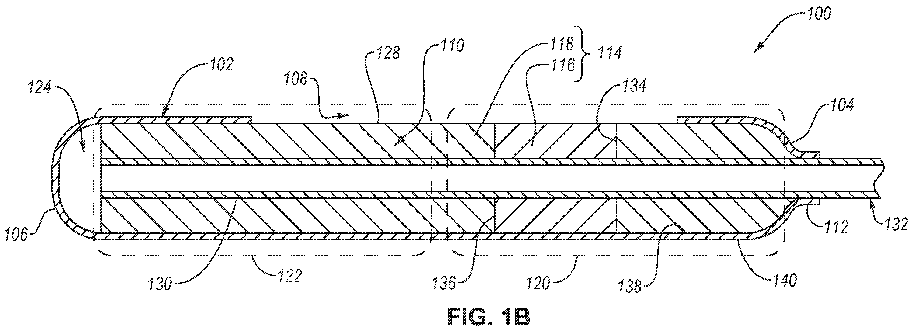

**FIG. 1A** is a top plan view of a fluid collection assembly 100, according to an embodiment. **FIG. 1B** is a cross-sectional schematic of the fluid collection assembly 100 taken along plane 1B-1B, according to an embodiment. The fluid collection assembly includes a fluid impermeable layer 102 that includes a proximal end region 104 and a distal end region 106. The fluid impermeable layer 102 at least defines at least one opening 108, a chamber 110, and a fluid outlet 112. The fluid collection assembly 100 also includes a porous medium 114 disposed in the chamber 110. The porous medium 114 includes a first porous material 116 and a second porous material 118. The first porous material 116 exhibits a first fluid permeability and a first compressibility and the second porous material 118 exhibits a second fluid permeability and a second compressibility. At least one of the first fluid permeability is greater than the second fluid permeability or the first compressibility is less than the second compressibility.

The different fluid permeabilities and/or compressibilities of the first and second porous materials 116, 118 cause the porous medium 114 to include a proximal zone 120 and a distal zone 122. The proximal zone 120 may include portions of the porous medium 114 that are configured to contact or otherwise be positioned adjacent to the urethral opening while the distal zone 122 may include portions of the porous medium 114 that are unlikely to contact or otherwise be positioned adjacent to the urethral opening. In other words, the proximal zone 120 is more likely that the distal zone 122 to receive large quantities of bodily fluids from the urethral opening. As such, the ability of the proximal zone 120 to receive the bodily fluids and flow the bodily fluids therethrough quickly may be more important than receiving the bodily fluids quickly into the distal zone 122. Meanwhile, the distal zone 122 may be configured to be more comfortable against the vaginal region since receiving the bodily fluids quickly therein is not prioritized. Thus, first and second porous materials 116, 118 may be disposed in the porous medium 114 such that proximal zone 120 exhibits at least one of a fluid permeability that is greater than or compressibility that is less than the distal zone 122.

Generally, the proximal zone 120 is relatively closer to the proximal end region 104 of the fluid impermeable layer 102 than the distal zone 122. As such, the proximal zone 120 may extend a distance from or near the proximal end region 104 of the fluid impermeable layer 102. Generally, the distal zone 122 is relatively closer to the distal end region 106 of the fluid impermeable layer 102 than the proximal zone 120. As such, the distal zone 122 may extend a distance from or near the distal end region 106 of the fluid impermeable layer 102 (*e.g.,* from or near the reservoir 124). In an embodiment, the distal zone 122 extends from or near the distal end region 106 to the proximal zone 120. In an embodiment, the proximal and distal zones 120, 122 may refer to distal and proximal halves of the porous medium 114.

The first porous material 116 extends from a portion an outer surface 128 of the porous medium 114. For example, the first porous material 116 may extend from at least a portion of an outer surface 128 of the proximal zone 120. As such, the first porous material 116 may directly abut, be positioned adjacent to, or otherwise positioned proximate to the urethral opening of the individual which allows the porous medium 114 to quickly receive a larger quantity of bodily fluids than if the first porous material 116 did not extend from the outer surface 128.

In an embodiment, as shown, the first porous material 116 extends from the outer surface 128 and through the porous medium 114. When the porous medium 114 defines a bore configured to receive a conduit 132 (as shown), the first porous material 116 may extend from the outer surface 128 to an inner surface 130 of the porous medium 114 that defines the bore. Extending the first porous material 116 through the porous medium 114 may facilitate formation of the porous medium 114. In an embodiment, the porous medium 114 may be formed by providing the second porous material 118, such as providing a generally hollow cylindrical second porous material 118. A cutout may be formed in the second porous material 118 and removed from the second porous material 118. The cutout may extend completely through the second porous material 118 which allows the cutout to be stamped or otherwise easily cut from the second porous material 118. The first porous material 116 may exhibit a size and shape that corresponds to the size and shape of the cutout. Thus, the first porous material 116 may be positioned in the cutout to form the porous medium 114. When the porous medium 114 is formed using such a method, the first porous material 116 may include one or more first lateral surfaces 134 and the second porous material 118 may include one or more second lateral surfaces 136. The second lateral surfaces 136 may completely enclose the first lateral surfaces 134.

In an embodiment, as previously discussed, the first porous material 116 may exhibit a first fluid permeability and the second porous material 118 may exhibit a second fluid permeability that is less than the first fluid permeability. The first fluid permeability may be greater than the second fluid permeability because the first porous material 116 exhibits at least one of a pores per cm (PPcm), density, or hydrophilicity that is different than the second porous material 118. The greater fluid permeability of the first porous material 116 compared to the second porous material may allow the first porous material 116 to receive bodily fluids quicker than the second porous material 118. Also, the greater fluid permeability of the first porous material 116 compared to the second porous material 118 may allow the vacuum (*e.g.*, air flow due to the vacuum) to preferentially travel in the first porous material 116 than the second porous material 118. Meanwhile, the second porous material 118 may be smoother, more compressible, or otherwise more comfortable against the vaginal region of the individual than the first porous material 116 since the fluid permeability of the second porous material 118 is not prioritized over comfort. Referring to the illustrated embodiment, as previously discussed, the first porous material 116 is configured to be positioned adjacent or otherwise proximate to the urethral opening of the individual. Thus, the first porous material 116 initially receives the bodily fluids discharged from the urethral opening. The relatively high fluid permeability of the first porous material 116 allows the bodily fluids to be quickly received into the porous medium 114 thereby preventing the bodily fluids from leaking. Further, the relatively high fluid permeability of the first porous material 116 allows the vacuum to be preferentially delivered to the first porous material 116 relative to at least some of the portions of the second porous material 118 about the first porous material 116, especially the portions of the second portions material 118 between the first porous material 116 and the proximal end region 104. Preferentially delivering the vacuum to the first porous material 116 increases the rate at which the bodily fluids are received into the first porous material 116, flow through the first porous material 116, and flow from the first porous material 116 to the second porous material 118. However, the first porous material 116 may exhibit a rougher surface or be otherwise less comfortable against the vaginal region of the individual than the second porous material 118. The presence of the second porous material limits the portions of the vaginal region that are exposed to the less comfortable first porous material 116 thereby making the fluid collection assembly 100 more comfortable to use.

In an embodiment, the first porous material 116 may exhibit a first compressibility and the second porous material 118 may exhibit a second compressibility that is greater than the first fluid permeability. The first compressibility may be less than the second compressibility because the first porous material 116 exhibits at least one of a PPcm, average fiber diameter, Young's modulus (*i.e.,* modulus of elasticity), yield or ultimate tensile strength, fiber entanglement or density a that is different than the second porous material 118. The lower compressibility of the first porous material 116 compared to the second porous material 118 may better inhibit the collapse of the passageways in the first porous material 116 than the second porous material 118. Inhibiting the collapse of the passageways of the first porous material 116 may allow better fluid flow. Meanwhile, the higher compressibility of the second porous material 118 compared to the first porous material 116 allows the second porous material 118 to better conform to the shape of the vaginal region and better distribute any pressure applied to the vaginal region, both of which improve comfort. Referring to the illustrated embodiment, as previously discussed, the first porous material 116 is configured to be positioned adjacent or otherwise proximate to the urethral opening of the individual. Thus, the first porous material 116 initially receives the bodily fluids discharged from the urethral opening. The prevention of the collapse of the passageways in the first porous material 116 allows the bodily fluids to be quickly received into the porous medium 114 and preferentially delivers the vacuum to the first porous material 116. However, the first porous material 116 may have difficultly conforming to the shape of the vaginal region and/or may have difficultly distributing pressure uniformly to the vaginal region. The presence of the second porous material 118 limits the portions of the vaginal region that are exposed to the less comfortable first porous material 116 thereby making the fluid collection assembly 100 more comfortable to use.

In the following embodiment, the PPI will be used in place of PPcm. 1PPI = 0.394 PPcm. In an embodiment, the first porous material 116 may exhibit a PPI is greater than a PPI exhibited by the second porous material 118. Generally, increasing the PPI of a material may increase the number of pores in the material, which may increase the rate at which bodily fluids and the vacuum may flow therethrough. As such, the first porous material 116 may exhibit a PPI that is greater than the second porous material. Also, increasing the PPI may increase the compressibility of the material by decreasing the solid content of the material and increase the surface roughness. The first and second porous materials 116, 118 may be independently selected to exhibit a PPI of about 10 PPI or greater, about 15 PPI or greater, about 20 PPI or greater, about 25 PPI or greater, about 30 PPI or greater, about 35 PPI or greater, about 40 PPI or greater, about 50 PPI or greater, about 60 PPI or greater, about 75 PPI or greater, about 100 PPI or greater, or in ranges of about 10 PPI to about 20 PPI, about 15 PPI to about 25 PPI, about 20 PPI to about 30 PPI, about 25 PPI to about 35 PPI, about 30 PPI to about 40 PPI, about 35 PPI to about 50 PPI, about 40 PPI to about 60 PPI, about 50 PPI to about 75 PPI, or about 60 PPI to about 100 PPI. The PPI of the first and second porous materials 116, 118 may be selected based on the desired fluid permeability and/or compressibility thereof.

In an embodiment, the first porous material 116 may exhibit a density that is less than a density exhibited by the second porous material 118. Generally, decreasing the density of a material may increase the porosity of the material which may increase the rate at which bodily fluids and at which the vacuum may flow therethrough. Also, decreasing the density of a material may increase the compressibility of the material by decreasing the solid content of the material and increase the surface roughness of the material. The first and second porous materials 116, 118 may be independently selected to exhibit a density of that is about 0.8 grams per centimeter cubed (g/cm3 will be here indicated as g/cc) or less, about 0.7 g/cc or less, about 0.65 g/cc or less, about 0.6 g/cc or less, about 0.55 g/cc or less, about 0.5 g/cc or less, about 0.45 g/cc or less, about 0.4 g/cc or less, about 0.35 g/cc or less, about 0.3 g/cc or less, about 0.25 g/cc or less, about 0.2 g/cc or less, about 0.15 g/cc or less, about 0.1 g/cc or less, about 0.075 g/cc or less, about 0.05 g/cc or less, about 0.04 g/cc or less, about 0.03 g/cc or less, about 0.02 g/cc or less, about 0.015 g/cc or less, about 0.01 g/cc or less, about 0.0075 g/cc or less, or in ranges of about 0.0075 g/cc to about 0.015 g/mm, about 0.01 g/cc to about 0.02 g/cc, about 0.015 g/mm to about 0.03 g/cc, about 0.02 g/cc to about 0.04 g/cc, about 0.03 g/cc to about 0.05 g/cc, about 0.04 g/cc to about 0.075 g/cc, about 0.05 g/cc to about 0.1 g/cc, about 0.75 g/cc to about 0.15 g/cc, about 0.1 g/cc to about 0.2 g/cc, about 0.15 g/cc to about 0.25 g/cc, about 0.2 g/cc to about 0.3 g/cc, about 0.25 g/cc to about 0.35 g/cc, about 0.3 g/cc to about 0.4 g/cc, about 0.35 g/cc to about 0.45 g/cc, about 0.4 g/cc to about 0.5 g/cc, about 0.45 g/cc to about 0.55 g/cc, about 0.5 g/cc to about 0. 6 g/cc, about 0.55 g/cc to about 0.65 g/cc, about 0.6 g/cc to about 0.7 g/cc, or about 0.65 g/cc to about 0.8 g/cc. The density of the first and second porous materials 116, 118 may be selected to be about 0.1% to about 99% the theoretical maximum density thereof *(i.e.,* the density of the first and second porous materials 116, 118 if such materials had no pores), such as about 0.1% to about 0.5%, about 0.25 % to about 0.75%, about 0.5% to about 1%, about 0.75% to about 1.5%, about 1% to about 2%, about 1.5% to about 2.5%, about 2% to about 3%, about 2.5% to about 3.5%, about 3% to about 4%, about 3.5% to about 5%, about 4% to about 6%, about 5% to about 7.5%, about 7% to about 10%, about 9% to about 12%, about 10% to about 15%, about 12.5 % to about 20%, about 15% to about 25%, about 20% to about 40%, about 30% to about 50%, about 40% to about 65%, or about 60% to about 99%. The density of the first and second porous materials 116, 118 may be selected based on the desired fluid permeability and/or compressibility thereof. The density of the first and second porous materials 116, 118 may also be selected based on the desired PPI thereof.

In an embodiment, the first porous material 116 may exhibit a percent porosity that is greater than the percent porosity exhibited by the second porous material 118. The greater percent porosity of the first porous material 116 may indicate that the first porous material 116 exhibit larger and/or greater quantity of pores that may quickly receive the bodily fluids than the second porous material 118. The percent porosity of the first and second porous materials 116, 118 may be independently selected to be about 1% to about 20 %, about 10% to about 30%, about 20% to about 40%, about 30% to about 50%, about 40% to about 55%, about 50% to about 60%, about 55% to about 65%, about 60% to about 70%, about 65% to about 75%, about 70% to about 80%, about 75% to about 82.5%, about 80% to about 85%, about 82.5% to about 87.5%, about 85% to about 90%, about 87.5% to about 92.5%, about 90% to about 95%, about 92.5% to about 97.5%, or about 95% to about 99%. The percent porosity of the first and second porous materials 116, 118 may be selected based on the desired fluid permeability, surface roughness, and compressibility thereof. The percent porosity of the first and second porous materials 116, 118 may depend, in part, on the density of the first and second porous materials 116, 118.

In an embodiment, the first porous material 116 may exhibit a hydrophilicity that is greater than a hydrophilicity exhibited by the second porous material 118. In other words, the first porous material 116 may exhibit a contact angle with water (a major constituent of bodily fluids) that is less than the contact angle formed between the second porous material 118 and water. Generally, increasing the hydrophilicity increases the ability of the material to pull the bodily fluids into the material. However, increasing the hydrophilicity of the material also increases the difficulty of removing the bodily fluids therefrom. The first and second porous materials 116, 118 may be independently selected to exhibit a contact angle with that is about 0° to about 10°, about 5° to about 15°, about 10° to about 20°, about 15° to about 25°, about 20° to about 30°, about 25° to about 35°, about 30° to about 40°, about 35° to about 45°, about 40° to about 50°, about 45° to about 55°, about 50° to about 60°, about 55° to about 65°, about 60° to about 70°, about 65° to about 75°, about 70° to about 80°, about 75° to about 85°, about 80° to about 90°, about 85° to about 95°, about 90° to about 100°, about 95° to about 105°, about 100° to about 120°, about 115° to about 125°, about 120° to about 130°, about 125° to about 135°, about 130° to about 140°, about 135° to about 145°, about 140° to about 50°, about 145° to about 155°, about 150° to about 160°, about 155° to about 165°, about 160° to about 170°, about 165° to about 175°, or about 170° to about 180°. The hydrophilicity (*i.e.,* contact angle with water) of the first and second porous materials 116, 118 may be selected based on the materials forming the first and second porous materials 116, 118. In an example, the first porous material 116 may be formed from a material exhibiting a hydrophilicity that is less than *(i.e.,* a contact angle with water that is greater than) a material forming the second porous material 118. In an example, the first porous material 116 may be at least partially coated with a material that increases the hydrophilicity thereof (*e.g.*, decreases the contact angle with water) and/or the second porous material 118 may be at least partially coated with a material that decreases a hydrophilicity thereof.

In an embodiment, the first porous material may exhibit an average fiber diameter that is greater than an average fiber diameter exhibited by the second porous material. Generally increasing the average fiber diameter increases the forces that the material may withstand without significant bending which, in turn, decreases the compressibility of the material. The first and second porous materials 116, 118 may be independently selected to exhibit an average fiber diameter that is about 0.1 µm to about 0.2 µm, about 0.15 µm to about 0.25 µm, about 0.2 µm to about 0.3 µm, about 0.25 µm to about 0.35 µm, about 0.3 µm to about 0.4 µm, about 0.35 µm to about 0.5 µm, about 0.4 µm to about 0.6 µm, about 0.5 µm to about 0.7 µm, about 0.6 µm to about 0.8 µm, about 0.7 µm to about 0.9 µm, about 0.8 µm to about 1 µm, about 0.9 µm to about 1.25 µm, about 1 µm to about 1.5 µm, about 1.25 µm to about 2 µm, about 1.5 µm to about 2.5 µm, about 2 µm to about 3 µm, about 2.5 µm to about 4 µm, about 3 µm to about 5 µm, about 4 µm to about 6 µm, about 5 µm to about 7 µm, about 6 µm to about 8 µm, about 7 µm to about 9 µm, about 8 µm to about 10 µm, about 9 µm to about 12.5 µm, about 10 µm to about 15 µm, about 12.5 µm to about 20 µm, about 15 µm to about 25 µm, about 20 µm to about 30 µm, about 25 µm to about 40 µm, about 30 µm to about 50 µm, about 40 µm to about 60 µm, about 50 µm to about 70 µm, about 60 µm to about 80 µm, about 70 µm to about 90 µm, about 80 µm to about 100 µm, about 90 µm to about 125 µm, about 100 µm to about 150 µm, about 125 µm to about 200 µm, about 150 µm to about 250 µm, about 200 µm to about 300 µm, about 250 µm to about 400 µm, or about 300 µm to about 500 µm. The average fiber diameter may be selected based on the desired compressibility of the first and second porous material 116, 118 since increasing the average fiber diameter decreases the compressibility of a material and *vice versa.* It is noted that the average fiber diameter may affect the PPI and/or the density of the first and second porous material 116, 118. For example, increasing the average fiber diameter may decrease the PPI and/or the density. As such, the average fiber density may be selected based on the desired PPI and/or density.

In an embodiment, at least one of the first porous material may exhibit a Young's modulus that is greater than a Young's modulus exhibited by the first porous material, the first porous material may exhibit a yield strength that is greater than a yield strength exhibited by the second porous material, or the first porous material may exhibit an ultimate tensile strength that is greater than an ultimate tensile strength exhibited by the second porous material. Generally increasing the Young's modulus, yield strength, or ultimate tensile strength of the material decreases the compressibility of the material. The Young's modulus, yield strength, and ultimate tensile strength are material properties and, thus, the Young's modulus, yield strength, and ultimate tensile strength of the first and second porous material 116, 118 are dependent on the material that forms the first and second porous material 116, 118. In an embodiment, the first and second porous material 116, 118 may be independently selected to exhibit a Young's modulus that is about 0.2 gigapascals ("GPa") or greater, about 0.3 GPa or greater, about 0.5 GPa or greater, about 0.75 GPa or greater, about 1 GPa or greater, about 1.5 GPa or greater, about 2 GPa or greater, about 3 GPa or greater, about 4 GPa or greater, about 5 GPa or greater, about 6 GPa or greater, about 7 GPa or greater, about 8 GPa or greater, about 9 GPa or greater, about 10 GPa or greater, about 11 GPa or greater, about 12.5 GPa or greater, about 15 GPa or greater, about 20 GPa or greater, about 25 GPa or greater, about 30 GPa or greater, about 40 GPa or greater, about 50 GPa or greater, about 75 GPa or greater, about 100 GPa or greater, or in ranges of about 0.2 GPa to about 0.5 GPa, about 0.3 GPa to about 0.75 GPa, about 0.5 GPa to about 1 GPa, about 0.75 GPa to about 1.5 GPa, about 1 GPa to about 2 GPa, about 1.5 GPa to about 3 GPa, about 2 GPa to about 4 GPa, about 3 GPa to about 5 GPa, about 4 GPa to about 6 GPa, about 5 GPa to about 7 GPa, about 6 GPa to about 8 GPa, about 7 GPa to about 9 GPa, about 8 GPa to about 10 GPa, about 9 GPa to about 11 GPa, about 10 GPa to about 12.5 GPa, about 11 GPa to about 15 GPa, about 12.5 GPa to about 20 GPa, about 15 GPa to about 25 GPa, about 20 GPa to about 30 GPa, about 25 GPa to about 40 GPa, about 30 GPa to about 50 GPa, about 35 GPa to about 75 GPa, or about 50 GPa to about 100 GPa. In an embodiment, the first and second porous material 118, 118 may be independently selected to exhibit a yield strength or ultimate tensile strength that is about 3 megapascals ("MPa) or greater, about 5 MPa or greater, about 7.5 MPa or greater, about 10 MPa or greater, about 15 MPa or greater, about 20 MPa or greater, about 30 MPa or greater, about 40 MPa or greater, about 50 MPa or greater, about 60 MPa or greater, about 70 MPa or greater, about 80 MPa or greater, about 100 MPa or greater, about 125 MPa or greater, about 150 MPa or greater, about 200 MPa or greater, about 250 MPa or greater, about 300 MPa or greater, about 400 MPa or greater, about 500 MPa or greater, about 600 MPa or greater, about 700 MPa or greater, about 800 MPa or greater, about 1 GPa or greater, or in ranges of about 3 MPa to about 7.5 MPa, about 5 MPa to about 10 MPa, about 7.5 MPa to about 15 MPa, about 10 MPa to about 20 MPa, about 15 MPa to about 30 MPa, about 20 MPa to about 40 MPa, about 30 MPa to about 50 MPa, about 40 MPa to about 60 MPa, about 50 MPa to about 70 MPa, about 60 MPa to about 80 MPa, about 70 MPa to about 100 MPa, about 80 MPa to about 125 MPa, about 100 MPa to about 150 MPa, about 100 MPa to about 200 MPa, about 150 MPa to about 300 MPa, about 200 MPa to about 400 MPa, about 300 MPa to about 500 MPa, about 400 MPa to about 600 MPa, about 500 MPa to about 700 MPa, about 600 MPa to about 800 MPa, about 700 MPa to about 1 GPa.

In an embodiment, the first porous material 116 may exhibit a fiber entanglement that is greater than a fiber entanglement exhibited by the second porous material 118. Generally increasing the fiber entanglement of a material decreases the compressibility of the material. The fiber entanglement may be dependent, for example, on the average fiber length, the woven pattern used to form the material, and the non-woven technique used to form the material. As such, the first porous material 116 may at least one of exhibit an average fiber length that is greater than, exhibit a woven pattern that is different than, or formed using a non-woven technique that is different than the second porous material 118.

In an embodiment, the first and second porous materials 116, 118 may be formed from the same isotropic material. The isotropic material may exhibit a compressibility and/or fluid permeability that is different in different orientations. As such, the isotropic material of the first porous material 116 may exhibit an orientation that is different that the isotropic material of the second porous material 118.

The porous medium 114 is disposed in the chamber 110. The porous medium 114 may cover at least a portion (*e.g.,* all) of the opening 108. The porous medium 114 is exposed to the environment outside of the chamber 110 through the opening 108. In an embodiment, the porous medium 114 may be configured to wick any bodily fluids away from the opening 108, thereby preventing the bodily fluids from escaping the chamber 110. The permeable properties referred to herein may be wicking, capillary action, diffusion, or other similar properties or processes, and are referred to herein as "permeable" and/or "wicking." Such "wicking" and/or "permeable" properties may not include absorption of the bodily fluids into at least a portion of the porous medium 114. Put another way, substantially no absorption or solubility of the bodily fluids into the material may take place after the material is exposed to the bodily fluids and removed from the bodily fluids for a time. While no absorption or solubility is desired, the term "substantially no absorption" may allow for nominal amounts of absorption and/or solubility of the bodily fluids into the porous medium 114 (*e.g.*, absorbency), such as less than about 30 wt% of the dry weight of the porous medium 114, less than about 20 wt%, less than about 10 wt%, less than about 7 wt%, less than about 5 wt%, less than about 3 wt%, less than about 2 wt%, less than about 1 wt%, or less than about 0.5 wt% of the dry weight of the porous medium 114. The porous medium 114 may also wick the bodily fluids generally towards an interior of the chamber 110, as discussed in more detail below. In an embodiment, the porous medium 114 may include at least one absorbent or adsorbent material.

The porous medium 114 may be formed from any suitable porous material. Examples of material that may form the porous medium 114 *(e.g.,* may form the first and second porous materials 116, 118) include gauze (*e.g.,* a silk, linen, or cotton gauze), felt, cotton, wool, silk, another fabric, a porous polymer (*e.g.*, nylon, polyester, polyurethane, polyethylene, polypropylene, etc.) structure, an open cell foam, a spun polymer (*e.g.,* spun nylon fiber), paper, a nonwoven material, a woven material, or combinations thereof.

As previously discussed, the fluid collection assembly 100 may include a fluid impermeable layer 102. The fluid impermeable layer 102 at least partially defines a chamber 110 (*e.g.*, interior region) and an opening 108. For example, the interior surface(s) 138 of the fluid impermeable layer 102 at least partially defines the chamber 110 within the fluid collection assembly 100. The fluid impermeable layer 102 temporarily stores the bodily fluids in the chamber 110. The fluid impermeable layer 102 may be formed of any suitable fluid impermeable material(s), such as a fluid impermeable polymer (*e.g.*, silicone, polypropylene, polyethylene, polyethylene terephthalate, neoprene, a polycarbonate, etc.), a metal film, natural rubber, another suitable material, any other fluid impermeable material disclosed herein, or combinations thereof. As such, the fluid impermeable layer 102 substantially prevents the bodily fluids from passing through the fluid impermeable layer 102. In an example, the fluid impermeable layer 102 may be air permeable and fluid impermeable. In such an example, the fluid impermeable layer 102 may be formed of a hydrophobic material that defines a plurality of pores. At least one or more portions of at least an outer surface 140 of the fluid impermeable layer 102 may be formed from a soft and/or smooth material, thereby reducing chaffing.

The opening 108 provides an ingress route for bodily fluids to enter the chamber 110. The opening 108 may be defined by the fluid impermeable layer 102 such as by an inner edge of the fluid impermeable layer 102. For example, the opening 108 is formed in and extends through the fluid impermeable layer 102, from the outer surface 140 to the inner surface 138, thereby enabling bodily fluids to enter the chamber 110 from outside of the fluid collection assembly 100.

In some examples, the fluid impermeable layer 102 may define a fluid outlet 112 sized to receive the conduit 132. The at least one conduit 132 may be disposed in the chamber 110 via the fluid outlet 112. The fluid outlet 112 may be sized and shaped to form an at least substantially fluid tight seal against the conduit 132 or the at least one tube thereby substantially preventing the bodily fluids from escaping the chamber 110.

The porous medium 114 may at least substantially completely fill the portions of the chamber 110 that are not occupied by the conduit 132. In some examples, the porous medium 114 may not substantially completely fill the portions of the chamber 110 that are not occupied by the conduit 132. In such an example, the fluid collection assembly 100 includes the reservoir 124 disposed in the chamber 110.

The reservoir 124 is a substantially unoccupied portion of the chamber 110. The reservoir 124 may be defined between the fluid impermeable layer 102 and the porous medium 114 (*e.g.,* one or more of the first or second porous materials 116, 118). The bodily fluids that are in the chamber 110 may flow through the porous medium 114 (*e.g.,* one or more of the first or second porous materials 116, 118) to the reservoir 124. The reservoir 124 may retain of the bodily fluids therein.

The bodily fluids that are in the chamber 110 may flow through the porous medium 114 to the reservoir 124. The fluid impermeable layer 102 may retain the bodily fluids in the reservoir 124. While depicted in the distal end region 106, the reservoir 124 may be located in any portion of the chamber 110 such as the proximal end region 104. The reservoir 124 may be located in a portion of the chamber 110 that is designed to be located in a gravimetrically low point of the fluid collection assembly 100 when the fluid collection assembly 100 is worn.

In some examples (not shown), the fluid collection assembly 100 may include multiple reservoirs, such as a first reservoir that is located at the portion of the chamber 110 closest to the inlet of the conduit 132 (*e.g.,* distal end region 106) and a second reservoir that is located at the portion of the of the chamber 110 that is at or near proximal end region 104). In another example, the porous medium 114 is spaced from at least a portion of the conduit 132, and the reservoir 124 may be the space between the porous medium 114 and the conduit 132.

The conduit 132 may be at least partially disposed in the chamber 110. The conduit 132 may be used to remove the bodily fluids from the chamber 110. The conduit 132 includes at least one wall defining an inlet, an outlet (not shown) downstream from the inlet, and a passageway. The outlet of the conduit 132 may be operably coupled to a vacuum source, such as a vacuum pump for withdrawing fluid from the chamber 110 through the conduit 132. For example, the conduit 132 may extend into the fluid impermeable layer 102 from the proximal end region 104 and may extend to the distal end region 106 to a point proximate to the reservoir 124 therein such that the inlet is in fluid communication with the reservoir 124. The conduit 132 fluidly couples the chamber 110 with the fluid storage container (not shown) or the vacuum source (not shown).

The conduit 132 may extend through a bore in the porous medium 114. In an embodiment, the conduit 132 extends from the fluid outlet 112, through the bore, to a location that is proximate to the reservoir 124. In such an embodiment, the inlet may not extend into the reservoir 124 and, instead, the inlet may be disposed within the porous medium 114 or at a terminal end thereof. For example, an end of the conduit 132 may be coextensive with or recessed within the porous medium 114. In an embodiment, the conduit 132 is at least partially disposed in the reservoir 124 and the inlet may be extended into or be positioned in the reservoir 124. The bodily fluids collected in the fluid collection assembly 100 may be removed from the chamber 110 via the conduit 132.

Locating the inlet at or near a location expected to be the gravimetrically low point of the chamber 110 when worn by an individual enables the conduit 132 to receive more of the bodily fluids than if inlet was located elsewhere and reduce the likelihood of pooling (*e.g.,* pooling of the bodily fluids may cause microbe growth and foul odors). For instance, the bodily fluids in the porous medium 114 may flow in any direction due to capillary forces. However, the bodily fluids may exhibit a preference to flow in the direction of gravity, especially when at least a portion of the porous medium 114 is saturated with the bodily fluids. Accordingly, one or more of the inlet or the reservoir 124 may be located in the fluid collection assembly 100 in a position expected to be the gravimetrically low point in the fluid collection assembly 100 when worn by an individual, such as the distal end region 106.

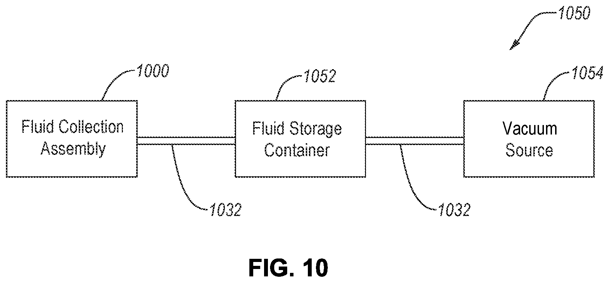

The inlet and the outlet of the conduit 132 are configured to fluidly couple (*e.g.,* directly or indirectly) the vacuum source (not shown) to the chamber 110 (*e.g.,* the reservoir 124). As the vacuum source (**FIG. 10**) applies a vacuum/vacuum in the conduit 132, the bodily fluids in the chamber 110 (*e.g.,* at the distal end region DER such as in the reservoir 124) may be drawn into the inlet and out of the fluid collection assembly 100 via the conduit 132. In some examples, the conduit 132 may be frosted or opaque (*e.g.,* black) to obscure visibility of the bodily fluids therein.

As previously discussed, the conduit 132 may be configured to be at least insertable into the chamber 110. In an example, the conduit 132 may be positioned in the chamber 110 such that a terminal end of the conduit 132 is spaced from the fluid impermeable layer 102 or other components of the fluid collection assembly 100 that may at least partially obstruct or block the inlet. Further, the inlet of the conduit 132 may be offset relative to a terminal end of the porous medium 114 such that the inlet is closer to the proximal end region 104 of the fluid collection assembly 100 than the terminal end of the porous medium 114. Offsetting the inlet in such a manner relative to the terminal end of the porous medium 114 allows the inlet to receive bodily fluids directly from the porous medium 114 and, due to hydrogen bonding, pulls more bodily fluids from the porous medium 114 into the conduit 132.+

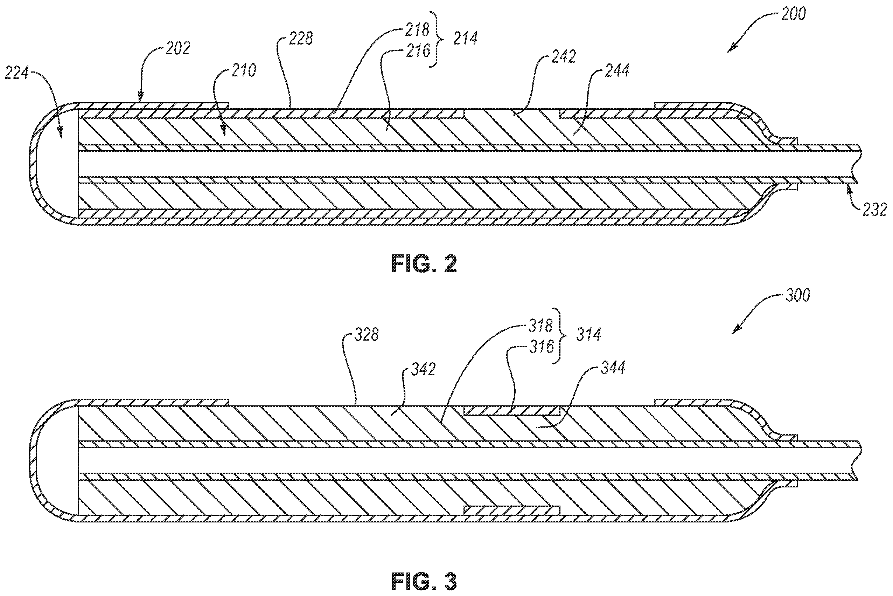

**FIGS. 2** and **3** are cross-sectional schematics for fluid collection assemblies that exhibit the same top plan view as the fluid collection assembly 100 illustrated in **FIG. 1A****,** according to different embodiments. Except as otherwise disclosed herein, the fluid collection assemblies illustrated in **FIGS. 2** and **3** are the same or substantially similar to any of the fluid collection assemblies disclosed herein. For example, the fluid collection assemblies illustrated in **FIGS. 2** and **3** may include a fluid impermeable layer defining a chamber and a porous medium disposed in the chamber. The porous medium may include a first porous material and a second porous material. The first porous material may exhibit at least one of a fluid permeability or a compressibility that is different than the second porous material. The first and second porous materials may be arranged such that the porous medium includes a proximal zone and a distal zone.

Referring to **FIG. 2****,** the first porous material 216 includes an outer portion 242 and an inner portion 244. The outer portion 242 extends inwardly from a portion of the outer surface 228 of the porous medium 214 that is configured to contact, be positioned adjacent to, or otherwise be positioned proximate to the urethral opening of an individual. The inner portion 244 may extend behind and support the second porous material 218 *(e.g.,* a fluid permeable membrane) and the outer portion 242. As such, the outer portion 242 may extend outwardly from the inner portion 244. During use, the outer portion 242 may receive a relatively large quantity of bodily fluids from the urethral opening. Regardless if the first porous material 216 exhibits a fluid permeability that is greater than or compressibility that is less than the second porous material 218, the bodily fluids may be received quickly into the outer portion 242 of the first porous material 216 due to the greater fluid permeability or uncollapsed passageways defined therein. The bodily fluids received by the outer portion 242 may then flow into the inner portion of the first porous material 216. Again, regardless if the first porous material 216 exhibits a fluid permeability that is greater than or compressibility that is less than the second porous material 218, the bodily fluids may quickly flow through the inner portion 244 to the reservoir 224 and/or the inlet of the conduit 232 due to the greater fluid permeability or uncollapsed passageways defined therein. In fact, the bodily fluids may flow through the porous medium 214 quicker than the porous medium 114 illustrated in FIG. 1B since at least some of the bodily fluids may flow through the porous medium 214 (*i.e.,* preferentially flow through the inner portion 244 of the first porous material 216) while avoiding the second porous material 218. Also, the vacuum may be more preferentially provided to the outer portion 242 of the first porous material 216 compared to the portion of the first porous material 116 shown in **FIG. 1B****,** since the vacuum will preferentially travel through the inner portion 244 of the first porous material 216 and the second porous material 218 will form a barrier that inhibits the vacuum leaking from the chamber 210 defined by the fluid impermeable layer 202.

The second porous material 218 still increases the comfort of fluid collection assembly 200 since it limits the portions of the vaginal region of the individual that contact the less comfortable first porous material 216. It is noted that the second porous material 218 may exhibit a decreases thickness measured perpendicular to a longitudinal axis of the fluid collection assembly 200 compared to the second porous material 118 illustrated in **FIG. 1B** since the inner portion 244 of the first porous material 216 extends behind the second porous material 218. When the second porous material 218 exhibits a compressibility that is greater than the first porous material 216, the decreased thickness of the second porous material 218 may slightly decrease the effect that the increased compressibility of the second porous material 218 has on improving the comfort of the fluid collection assembly 200.

The porous medium 214 may be formed by forming a cutout in the second porous material 218 and forming a protrusion (*i.e.*, the outer portion 242) in the first porous material 216 that corresponds to the cutout formed in the second porous material 218. The first porous material 216 may then be disposed within the second porous material 218 with the outer portion 242 disposed through the cutout. As such, the second porous material 218 may completely enclose the outer portion 242.

Referring to **FIG. 3****,** the second porous material 318 includes an outer portion 342 and an inner portion 344. The outer portion 342 extends inwardly from a portion of the outer surface 328 of the porous medium 314 that is not configured to contact, be positioned adjacent to, or otherwise be positioned proximate to the urethral opening of an individual. The inner portion 344 may extend behind and support the first porous material 316 *(e.g.,* fluid permeable membrane). As such, the outer portion 342 may extend outwardly from the inner portion 344. The second porous material 318 increases the comfort of fluid collection assembly 300 since it limits the portions of the vaginal region of the individual that contact the less comfortable first porous material 316. The second porous material 318 may define a recess adjacent to the inner portion 344. The first porous material 316 may be positioned in the recess. During use, the first porous material 316 may receive a relatively large quantity of bodily fluids from the urethral opening. Regardless if the first porous material 316 exhibits a fluid permeability that is greater than or compressibility that is less than the second porous material 318, the bodily fluids may be received quickly into the first porous material 316 due to the greater fluid permeability thereof or non-collapsed passageways defined therein. The bodily fluids received by the first porous material 316 may then flow into the second porous material 318.

The porous medium 314 may be formed by forming the recess in the second porous material 318 and forming (*e.g.*, shaping) the first porous material 316 to fit within the recess. The first porous material 316 may then be disposed within the recess defined by the second porous material 318. As such, the second porous material 318 may completely enclose the outer portion 342.

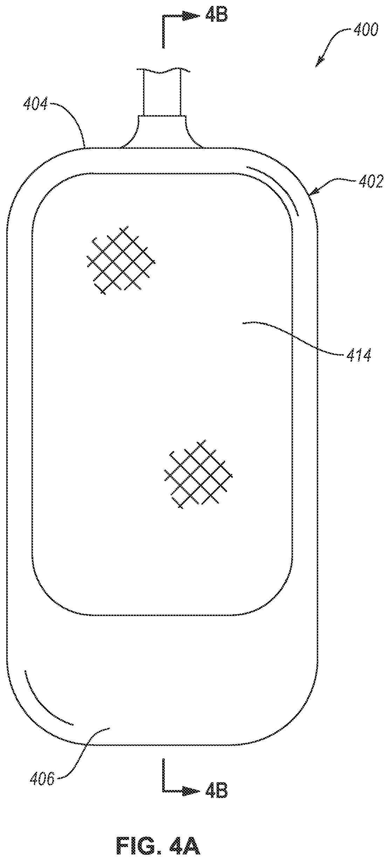

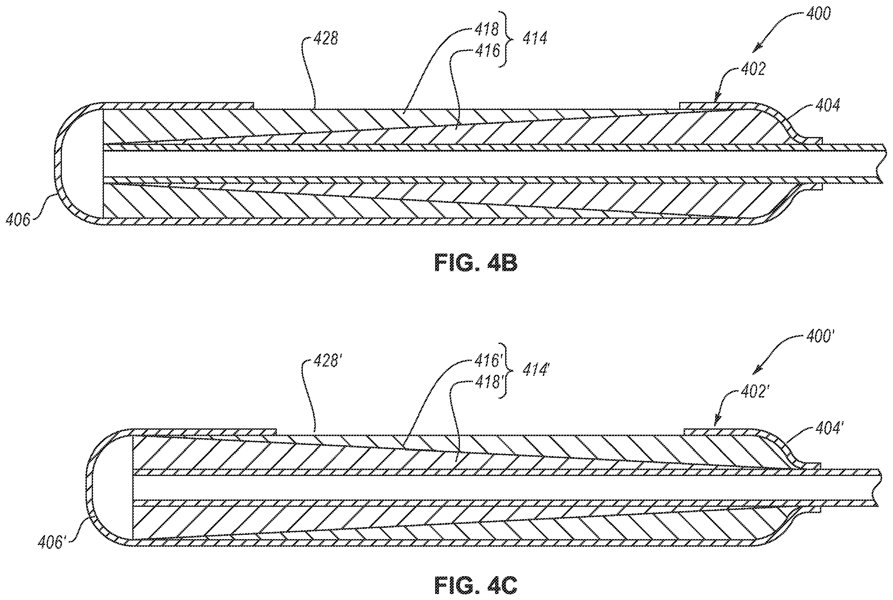

**FIG. 4A** is a top plan view of a fluid collection assembly 400, according to an embodiment. **FIG. 4B** is a cross-sectional schematic of the fluid collection assembly 400 taken along plane 4B-4B. Except as otherwise disclosed herein, the fluid collection assembly 400 is the same or substantially similar to any of the fluid collection assemblies disclosed herein. For example, the fluid collection assembly 400 includes a fluid impermeable layer 402 including a proximal end region 404 and a distal end region 406. The fluid collection assembly 400 also includes a porous medium 414 disposed in the fluid impermeable layer 402. The porous medium 414 includes a first porous material 416 and a second porous material 418.

The second porous material 418 forms all of the outer surface 428 of the porous medium 414, which makes all of the outer surface 428 that contacts the vaginal region of the individual more comfortable to use. However, the thickness of the second porous material 418 is tapered along at least a portion (*e.g.,* all) of a length of the porous medium 414 measured parallel to a longitudinal axis of the fluid collection assembly 400. For example, the thickness of the second porous material 418 decreases with increasing proximity to the proximal end region 404. Since the urethral opening is generally positioned proximate to the proximal end region 404, the decreased thickness of second porous material 418 may prevent or at least inhibit the second porous material 418 from being a significant obstacle to bodily fluids entering porous medium 414. The thickness of the second porous material 418 may increase with increasing proximity to the distal end region 406. As such, when the second porous material 418 exhibits a compressibility that is greater than the first porous material 416, the increased compressibility of the second porous material 418 may have a greater effect on the comfort of the fluid collection assembly 400 with increased proximity to the distal end region 406.

The first porous material 416 is spaced from the outer surface 428 of the porous medium 414 which, as previously discussed, may make the fluid collection assembly 400 more comfortable to use. However, the thickness of the first porous material 416 is tapered along at least a portion (*e.g.,* all) of the length of the porous medium 414 in a manner that is opposite the second porous material 418. For example, the thickness of the first porous material 416 may increase with increasing proximity to the proximal end region 404 and may decrease with increasing proximity to the distal end region 406. As such, the thickness of the first porous material 416 may be significantly greater than the thickness of the second porous material 418 at a location that is adjacent to the urethral opening of the individual during use. The increased thickness of the first porous material 416 may help pull bodily fluids through the second porous material 418 that is adjacent to the urethral opening since the first porous material 416 may exhibit a fluid permeability and/or more non-obstructed passageways compared to the second porous material 418. The variation of the thickness of the first porous material 416 may also facilitate a delivering the vacuum to a portion of the porous medium 414 that is proximate to the urethral opening. For example, as previously discussed, the vacuum preferentially flows through the first porous material 416. The increased thickness of the second porous material 418 proximate to the distal end region 406 inhibits the vacuum leaking from the porous medium 414 at a location that is proximate to the distal end region 406 *(i.e.,* spaced from the urethral opening). The decreased thickness of the second porous material 418 proximate to the proximal end region 404 causes more of the vacuum to leak from the porous medium 414 at a location that is proximate to the proximal end region 404 which helps pull the bodily fluids into the porous medium 414 (*e.g.,* pulls the bodily fluids through the second porous material 418 and into the first porous material 416).

**FIG. 4C** is a cross-sectional schematic of a fluid collection assembly 400', according to an embodiment. Except as otherwise disclosed herein, the fluid collection assembly 400' is the same or substantially similar to any of the fluid collection assemblies disclosed herein. For example, the fluid collection assembly 400' includes a fluid impermeable layer 402' including a proximal end region 404' and a distal end region 406'. The fluid collection assembly 400' also includes a porous medium 414' disposed in the fluid impermeable layer 402'. The porous medium 414' includes a first porous material 416' and a second porous material 418'.

The fluid collection assembly 400' is substantially similar to the fluid collection assembly 400 of **FIGS. 4A** and **4B** except that the first porous material 416' forms all of the outer surface 428' of the porous medium 414' instead of the second porous material 418'. Forming the entirety of the outer surface 428' from the first porous material 416' may make the fluid collection assembly 400' more uncomfortable than the fluid collection assembly 400'. However, forming the entirety of the outer surface 428' from the first porous material 416' prevents the second porous material 418' from being a barrier that impedes flow of the bodily fluids into the first porous material 416'. For example, bodily fluids discharged from the urethral opening are able to immediately contact the first porous material 416'. As such, the bodily fluids may flow more quickly into the first porous material 416' and, in turn, into the interior of the porous medium 414' due to the high fluid permeability of the first porous material 416' than if the second porous material 418' formed at least a portion of the porous medium 414'. As such, the fluid collection assembly 400' may be more effective at receiving bodily fluids than the first collection assembly 400 of **FIGS. 4A** and **4B****,** especially when the individual discharges bodily fluids at a relatively high rate.

Further, forming the entirety of the outer surface 428' from the first porous material 416' may facilitate using the fluid collection assembly 400' when the fluid collection assembly 400' is likely to move during use. For example, the fluid collection assembly 400' may use contact between the thighs of the individual and the fluid impermeable layer 402' to maintain the correct position of the fluid collection assembly 400' against the urethral opening. However, individuals who have thin thighs, are forgetful (*e.g.*, individuals with dementia, young children, etc.), or who often move may have difficulty maintaining sufficient contact between their thighs and the fluid impermeable layer 402' to maintain the position of the fluid collection assembly 400'. Thus, the fluid collection assembly 400' is likely to move when used with such individuals. Normally, allowing any fluid collection assembly to move relative to the individual increases the likelihood that the fluid collection assembly leaks bodily fluids because the bodily fluids contact an unexpected portion of the porous medium or a gap forms between the fluid collection assembly and the individual. However, forming the entirety of the outer surface 428' from the first porous material 416' decreases the amount of bodily fluids that may leak when the fluid collection assembly 400' is used with such individuals. For example, the high fluid permeability of the first porous material 416' quickly draws the bodily fluids into the porous medium 414' regardless of which portions of the porous medium 414' initially receive the bodily fluids. Further, forming the entirety of the outer surface 428' from the first porous material 416' ensures a greater percentage of any bodily fluids that contact the porous medium 414' are received into the porous medium 414' than if the second porous material 418' forms any portion of the outer surface 428'.

The thickness of the first porous material 416' is tapered along at least a portion *(e.g.,* all) of a length of the porous medium 414' measured parallel to a longitudinal axis of the fluid collection assembly 400'. For example, the thickness of the first porous material 416' increases with increasing proximity to the proximal end region 404'. As previously discussed, the increased thickness of first porous material 416' may facilitate receiving bodily fluids deeper into the porous medium 414'. The thickness of the second porous material 418' is tapered along at least a portion *(e.g.,* all) of the length of the porous medium 414 in a manner that is opposite the first porous material 416'. In an example, the second porous material 418' is more compressible than the first porous material 416'. In such an example, the decreased and increased thickness of the first porous material 416' and the second porous material 418', respectively, with increasing proximity to the distal end region 406' makes the overall compressibility of the porous medium 414' increase with increasing proximity to the distal end region 406'. In other words, the second porous material 418' is still able to improve the comfort of the fluid collection assembly 400' even though the second porous material 418' does not form part of the outer surface 428'.

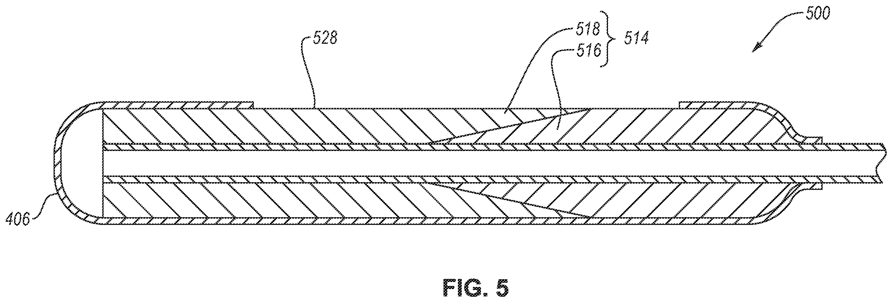

**FIG.** 5 is a cross-sectional schematic of a fluid collection assembly 500, according to an embodiment. The fluid collection assembly 500 is the same as the fluid collection assembly 400 illustrated in **FIGS. 4A-4B** except that the first and second porous materials 516, 518 are tapered along only a portion of a length of the porous medium 514. Tapering the first and second porous materials 516, 518 along only a portion of the length of the porous medium 514 may allow a portion of the first porous material 516 to form a portion of the outer surface 528 of the porous medium 514. For example, the first porous material 516 may form a portion of the outer surface 528 that is configured to be positioned adjacent to the urethral opening. As such, the portion of the outer surface 528 that is formed by the fluid porous material 516 may allow for improved fluid into the porous medium 514 compared to the porous medium 414 illustrated in **FIG. 4B** since the second porous material 518 may not form a barrier for bodily fluids entering the first porous material 516. It is noted that the first and second porous materials 516, 518 may be tapered in the same direction as illustrated in **FIG. 4C** instead of the direction illustrated in **FIG. 5**

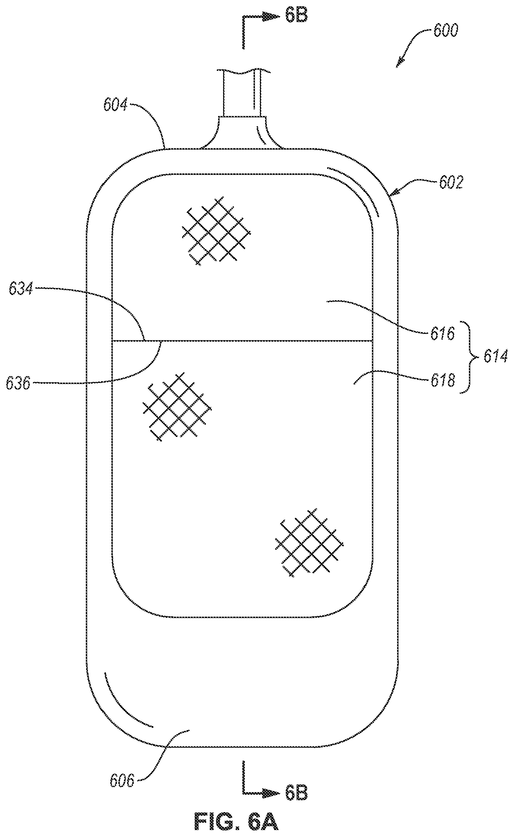

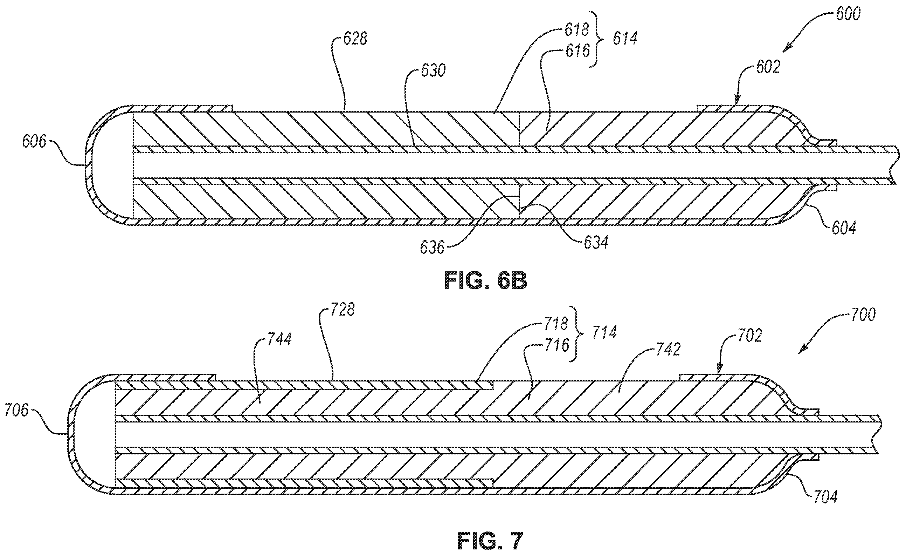

**FIG. 6A** is a top plan view of a fluid collection assembly 600, according to an embodiment. **FIG. 6B** is a cross-sectional schematic of the fluid collection assembly 600, according to an embodiment. Except as otherwise disclosed herein, the fluid collection assembly 600 is the same or substantially similar to any of the fluid collection assemblies disclosed herein. For example, the fluid collection assembly 600 includes a fluid impermeable layer 602 including a proximal end region 604 and a distal end region 606. The fluid collection assembly 600 also includes a porous medium 614 disposed in the fluid impermeable layer 602. The porous medium 614 includes a first porous material 616 and a second porous material 618.

The first and second porous materials 616, 618 form district portions of the porous medium 614 such that the first porous material 616 forms the proximal zone of the porous medium 614 and the second porous material 618 forms the distal zone of the porous medium 614. For example, each of the first and second porous materials 616, 618 may form a portion of the outer surface 628 of the porous medium 614. Each of the first porous material 616 and the second porous material 618 may include one or more first lateral surfaces 634 and one or more second lateral surfaces 636, respectively, which extend from the outer surface 628 through the porous medium 614 *(e.g.,* extend from the outer surface 628 to the inner surface 630 that defines a bore). In an embodiment, the first and second porous materials 616, 618 may exhibit a shape that corresponds to the overall shape of the porous medium 614 except that the first and second porous materials 616, 618 exhibit a shorter length. For example, when the porous medium 614 exhibits a generally cylindrical shape, the first and second porous materials 616, 618 may exhibit a generally cylindrical shape.

The porous medium 614 may be easier to manufacture than the other porous mediums disclosed herein. For example, the porous medium 614 may be formed without forming a cutout, forming a recess, forming a protrusion, or positioning one porous material on or in another porous material. Instead, the porous medium 614 may be formed by providing the first and second porous materials 616, 618 and, optionally, adjusting the lengths of the first and second porous materials. The first and second porous materials 616, 618 may then be positioned adjacent to each other to form the porous medium 614.

**FIG. 7** is a cross-sectional schematic of a fluid collection assembly 700, according to an embodiment. It is noted that the fluid collection assembly 700 may exhibit the same top plan view as the fluid collection assembly 600 illustrated in **FIG. 6A****.** Except as otherwise disclosed herein, the fluid collection assembly 700 is the same or substantially similar to any of the fluid collection assemblies disclosed herein. For example, the fluid collection assembly 700 includes a fluid impermeable layer 702 including a proximal end region 704 and a distal end region 706. The fluid collection assembly 700 also includes a porous medium 714 disposed in the fluid impermeable layer 702. The porous medium 714 includes a first porous material 716 and a second porous material 718.

Similar to the porous medium 214 of **FIG. 2****,** the first porous material 716 includes an outer portion 742 and an inner portion 744. The outer portion 742 extends inwardly from a portion of the outer surface 728 of the porous medium 714 that is configured to contact, be positioned adjacent to, or otherwise be positioned proximate to the urethral opening of an individual. The inner portion 744 may extend behind the second porous material 718 and the outer portion 742. As such, the outer portion 742 may extend outwardly from the inner portion 744. The second porous material 718 still increases the comfort of fluid collection assembly 700 since it limits the portions of the vaginal region of the individual that contact the less comfortable first porous material 716.

The porous mediums disclosed herein may include at least one intermediate porous material (*e.g.*, a third porous material, a fourth porous material, etc.) in addition to the first and second porous materials discussed above. The intermediate porous material may exhibit an intermediate fluid permeability and an intermediate compressibility. In an embodiment, the intermediate fluid permeability of the intermediate porous material may be less than and greater than the first fluid permeability of the first porous material and the second fluid permeability of the second porous material, respectively. In an embodiment, the intermediate compressibility of the intermediate porous material may be greater than and less than the first compressibility of the first porous material and the second compressibility of the second porous material, respectively. The intermediate porous material may be positioned between or otherwise contact one or more of the first porous material or the second porous material. The intermediate porous materials may allow for greater control of the fluid permeability and compressibility of the porous medium than if the porous medium only included the first and second porous materials. The intermediate porous materials may form part of the proximal zone of the porous medium, a distal zone of the porous medium, or an intermediate zone of the porous medium that is between the proximal and distal zones.

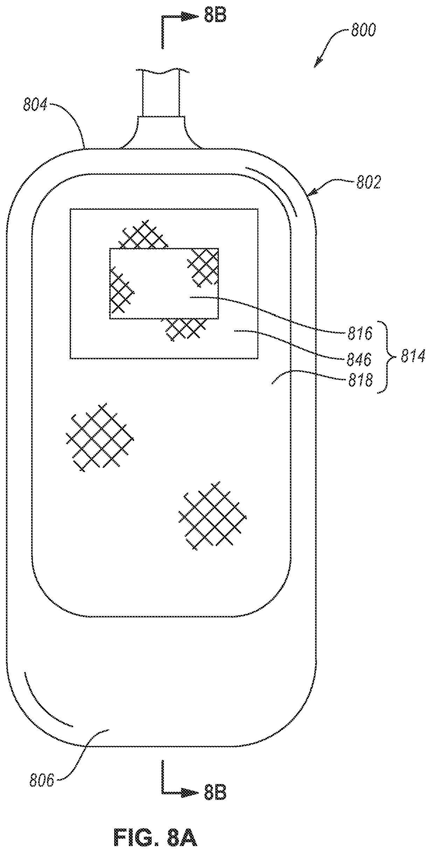

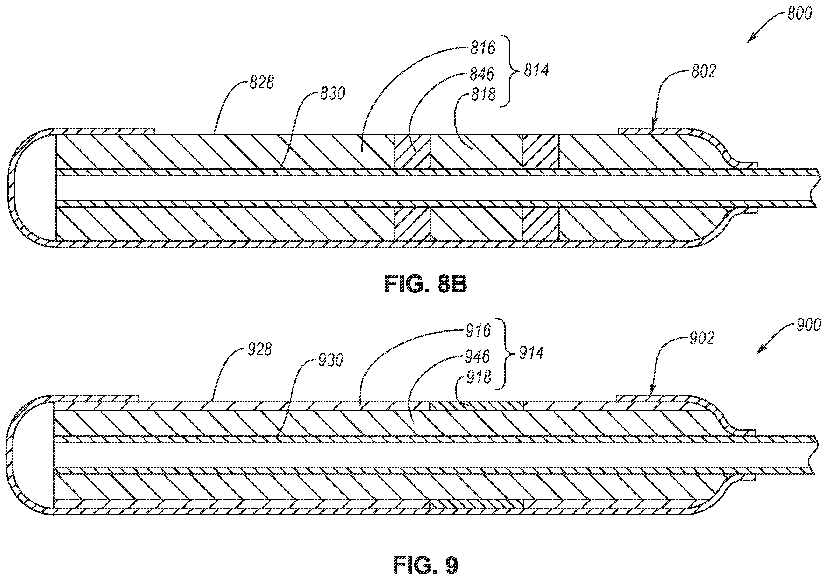

**FIG. 8A** is a top plan view of a fluid collection assembly 800 including a porous medium 814 that has a third porous material 846 in addition to the first porous material 816 and the second porous material 818, according to an embodiment. **FIG. 8B** is a cross-sectional schematic of the fluid collection assembly 800 taken along plane 8B-8B. Except as otherwise disclosed herein, the fluid collection assembly 800 is the same or substantially similar to any of the fluid collection assemblies disclosed herein. For example, the fluid collection assembly 800 includes a fluid impermeable layer 802 and a porous medium 814 disposed in the fluid impermeable layer 802. The porous medium 814 includes a first porous material 816 and a second porous material 818.

Similar to the fluid collection assembly 100 illustrated in **FIG. 1B****,** the first porous material 816 and the second porous material 818 extend from the outer surface 828 of the porous medium 814 and through the porous medium 814 *(e.g.,* to an inner surface 830). The third porous material 846 is positioned between the first and second porous material 816 and extends from the outer surface 828 of the porous medium 814 and through the porous medium 814. In an embodiment, the third porous material 846 may facilitate operation of the fluid collection assembly 800 when the fluid collection assembly 800 is misplaced or mispositioned. For example, the fluid collection assembly 800 is configured such that the urethral opening of the individual is positioned adjacent to the urethral opening. However, the fluid collection assembly 800 may be misplaced on the individual such that the urethral opening is not positioned adjacent to the urethral opening. When the fluid collection assembly 800 is misplaced, the urethral opening may be positioned adjacent to the third porous material 846 and, thus, the bodily fluids may be received into the porous medium 814 better than if the urethral opening was positioned adjacent to the second porous material 818 (which is what may occur if the porous medium 814 did not include the third porous material 846). Further, the third porous material 846 may be more comfortable than the first porous material 816 and, thus, the third porous material 846 may make the fluid collection assembly 800 more comfortable than if the third porous material 846 was instead formed from the first porous material 816.

**FIG. 9** is a schematic cross-sectional schematic of a fluid collection assembly 900 including a porous medium 914 that has a third porous material 946 in addition to the first porous material 916 and the second porous material 918, according to an embodiment. Except as disclosed herein, the fluid collection assembly 900 may be the same or substantially similar to any of the fluid collection assemblies disclosed herein. For example, the fluid collection assembly 900 may be the same or substantially similar to the fluid collection assembly 200 illustrated in **FIG. 2** except that the outer portion 242 and the inner portion 244 are formed from different porous materials. In an embodiment, as illustrated, the fluid collection assembly 900 include a fluid impermeable layer 902 and a porous medium 914 disposed therein. The porous medium 914 includes a first porous material 916 and a second porous material 918 that form a part of the outer surface 928 of the porous medium 914 (*i.e.,* the first porous material 916 forms the outer portion). The porous medium 914 also includes a third porous material 946 extending behind at least a portion of the first and second porous materials 916, 918 (*i.e.,* the third porous material 946 forms the inner portion). The first porous material 916 may facilitate bodily fluids entering the porous medium 914 and the second porous material 918 may make the fluid collection assembly 900 more comfortable. Compared to the fluid collection assembly 200, the third porous material 946 may improve the compressibility of the porous medium 914 while minimizing any decrease in the overall fluid permeability of the porous medium 914. In an embodiment, not shown, the third porous material 946 forms the outer portion and the first porous material 916 forms the inner portion. In such an embodiment, the third porous material 946 may make the outer surface 928 smoother or otherwise more comfortable and the first porous material 916 may improve fluid flow through the porous medium 914 than the embodiment illustrated in **FIG. 9****.**

It is noted that any of the other embodiments disclosed herein may include an intermediate porous material.

**FIG. 10** is a block diagram of a fluid collection system 1050 for fluid collection, according to an embodiment. The fluid collection system 1050 includes a fluid collection assembly 1000, a fluid storage container 1052, and a vacuum source 1054. The fluid collection assembly 1000 may be the same or substantially similar to any of the fluid collection assemblies disclosed herein. The fluid collection assembly 1000, the fluid storage container 1052, and the vacuum source 1054 may be fluidly coupled to each other via one or more conduits 1032. For example, fluid collection assembly 1000 may be operably coupled to one or more of the fluid storage container 1052 or the vacuum source 1054 via the conduit 1032. The bodily fluids collected in the fluid collection assembly 1000 may be removed from the fluid collection assembly 1000 via the conduit 1032 which protrudes into the fluid collection assembly 1000. For example, an inlet of the conduit 1032 may extend into the fluid collection assembly 1000, such as to a reservoir therein. The outlet of the conduit 1032 may extend into the fluid collection assembly 1000 or the vacuum source 1054. Vacuum force may be introduced into the chamber of the fluid collection assembly 1000 via the inlet of the conduit 1032 responsive to vacuum (e.g., vacuum) force applied at the outlet of the conduit 1032.

The vacuum force may be applied to the outlet of the conduit 1032 by the vacuum source 1054 either directly or indirectly. The vacuum force may be applied indirectly via the fluid storage container 1052. For example, the outlet of the conduit 1032 may be disposed within the fluid storage container 1052 and an additional conduit 1032 may extend from the fluid storage container 1052 to the vacuum source 1054. Accordingly, the vacuum source 1054 may apply vacuum to the fluid collection assembly 1000 via the fluid storage container 1052. The vacuum force may be applied directly via the vacuum source 1054. For example, the outlet of the conduit 1032 may be disposed within the vacuum source 1054. An additional conduit 1032 may extend from the vacuum source 1054 to a point outside of the fluid collection assembly 1000, such as to the fluid storage container 1052. In such examples, the vacuum source 1054 may be disposed between the fluid collection assembly 1000 and the fluid storage container 1052.

The fluid storage container 1052 is sized and shaped to retain bodily fluids therein. The fluid storage container 1052 may include a bag (e.g., drainage bag), a bottle or cup (e.g., collection jar), or any other enclosed container for storing bodily fluids such as urine. In some examples, the conduit 1032 may extend from the fluid collection assembly 1000 and attach to the fluid storage container 1052 at a first point therein. An additional conduit 1032 may attach to the fluid storage container 1052 at a second point thereon and may extend and attach to the vacuum source 1054. Accordingly, a vacuum (e.g., vacuum) may be drawn through fluid collection assembly 1000 via the fluid storage container 1052. Bodily fluids, such as urine, may be drained from the fluid collection assembly 1000 using the vacuum source 1054.

The vacuum source 1054 may include one or more of a manual vacuum pump, and electric vacuum pump, a diaphragm pump, a centrifugal pump, a displacement pump, a magnetically driven pump, a peristaltic pump, or any pump configured to produce a vacuum. The vacuum source 1054 may provide a vacuum or vacuum to remove bodily fluids from the fluid collection assembly 1000. In some examples, the vacuum source 1054 may be powered by one or more of a power cord (e.g., connected to a power socket), one or more batteries, or even manual power (e.g., a hand operated vacuum pump). In some examples, the vacuum source 1054 may be sized and shaped to fit outside of, on, or within the fluid collection assembly 1000. For example, the vacuum source 1054 may include one or more miniaturized pumps or one or more micro pumps. The vacuum sources 1054 disclosed herein may include one or more of a switch, a button, a plug, a remote, or any other device suitable to activate the vacuum source 1054.

Terms of degree (*e.g.*, "about," "substantially," "generally," etc.) indicate structurally or functionally insignificant variations. In an example, when the term of degree is included with a term indicating quantity, the term of degree is interpreted to mean ± 10%, ±5%, or +2% of the term indicating quantity. In an example, when the term of degree is used to modify a shape, the term of degree indicates that the shape being modified by the term of degree has the appearance of the disclosed shape. For instance, the term of degree may be used to indicate that the shape may have rounded corners instead of sharp corners, curved edges instead of straight edges, one or more protrusions extending therefrom, is oblong, is the same as the disclosed shape, etc.

## Claims

1. A fluid collection assembly (100), comprising:
a fluid impermeable layer (102) including a proximal end region (104) and a distal end region (106), the fluid impermeable layer at least defining at least one opening (108), a chamber (110), and a fluid outlet (112); and
a porous medium (114) disposed in the chamber, the porous medium including:
a proximal zone (120) extending from or near the proximal end region of the fluid impermeable layer;
a distal zone (122) extending from or near the distal end region of the fluid impermeable layer to the proximal zone;
a first porous material (116) exhibiting a first fluid permeability and a first compressibility; and
a second porous material (118) exhibiting a second fluid permeability and a second compressibility;
wherein at least one of the first fluid permeability is greater than the second fluid permeability or the first compressibility is less than the second compressibility; and
**characterized in that**:
the first porous material and the second porous material are disposed in the porous medium such that at least one of:
the proximal zone exhibits a fluid permeability that is greater than the fluid permeability of the distal zone; or
the proximal zone exhibits a compressibility that is less than a compressibility of the distal zone.

2. The fluid collection assembly of claim 1, wherein the first fluid permeability is greater than the second fluid permeability and the proximal zone exhibits a fluid permeability that is greater than a fluid permeability exhibited by the distal zone.

3. The fluid collection assembly of claim 2, wherein at least one of:
the first porous material exhibits a pores per cm that is greater than a pores per cm exhibited by the second porous material; or
the first porous material exhibits a percent porosity that is greater than a percent porosity exhibited by the second porous material.

4. The fluid collection assembly of any one of claims 2 or 3, wherein at least one of:
the first porous material exhibits a density that is greater than a density exhibited by the second porous material; or
the first porous material exhibits hydrophilicity that is greater than a hydrophilicity exhibited by the second porous material.

5. The fluid collection assembly of any one of claims 2-4, wherein the porous medium includes at least one intermediate porous material, the at least one additional porous material exhibiting a fluid permeability that is less than the first fluid permeability and greater than the second fluid permeability.

6. The fluid collection assembly of any one of claims 1-5, wherein the first compressibility is less than the second compressibility and the proximal zone exhibits a compressibility that is less than a compressibility exhibited by the distal zone.

7. The fluid collection assembly of claim 6, wherein the first porous material exhibits an average fiber diameter that is greater than an average fiber diameter exhibited by the second porous material.

8. The fluid collection assembly of any one of claims 6 or 7, wherein at least one of:
the first porous material exhibits a pores per cm that is greater than a pores per cm exhibited by the second porous material;
the first porous material exhibits a Young's modulus that is greater than a Young's modulus exhibited by the second porous material;
the first porous material exhibits a yield strength that is greater than a yield strength exhibited by the second porous material; or
the first porous material exhibits a fiber entanglement that is greater than a fiber entanglement exhibited by the second porous material.

9. The fluid collection assembly of any one of claims 6-8, wherein the first porous material and the second porous material are formed from isotropic materials that are oriented differently.

10. The fluid collection assembly of any one of claims 1-9, wherein the porous medium exhibits an outer surface that is exposed through the at least one opening, the outer surface formed by the first porous material and the second porous material.

11. The fluid collection assembly of claim 10, wherein a portion of the first porous material extends behind at least a portion of the second porous material.

12. The fluid collection assembly of any one of claims 10 or 11, wherein the first porous material includes at least one lateral surface extending inwardly from the outer surface of the porous material, the second porous material completely encloses the at least one lateral surface of the first portion material.

13. The fluid collection assembly of any one of claims 1-9, wherein the porous medium exhibits an outer surface that is exposed through the at least one opening, the outer surface formed only by the first porous material.

14. The fluid collection assembly of any one of claims 1-13,
wherein a thickness of at least a portion of the first portion decreases along a direction extending from the proximal end region to the distal end region.

15. A fluid collection system, comprising:
the fluid collection assembly of any one of claims 1-14;
a fluid storage container (1052); and
a vacuum source (1054);
wherein the chamber of the fluid collection assembly, the fluid storage container, and the vacuum source are in fluid communication with each that, when one or more bodily fluids are present in the chamber, a vacuum provided from the vacuum source to the chamber of the fluid collection assembly removes the one or more bodily fluids from the chamber and deposits the bodily fluids in the fluid storage container.

## Patentansprüche

1. Flüssigkeitsammelvorrichtung (100), umfassend:
eine flüssigkeitsimpermeable Schicht (102) einschließlich eines proximalen Endbereichs (104) und eines distalen Endbereichs (106), wobei die flüssigkeitsimpermeable Schicht mindestens eine Öffnung (108), eine Kammer (110) und einen Flüssigkeitsauslass (112) definiert; und
ein poröses Medium (114), angeordnet in der Kammer, wobei das poröse Medium Folgendes einschließt:
eine proximale Zone (120), die sich von oder nahe dem proximalen Endbereich der flüssigkeitsimpermeablen Schicht erstreckt;
eine distale Zone (122), die sich von oder nahe dem distalen Endbereich der flüssigkeitsimpermeablen Schicht bis zu der proximalen Zone erstreckt;
ein erstes poröses Material (116), das eine erste Flüssigkeitspermeabilität und eine erste Komprimierbarkeit aufweist; und
ein zweites poröses Material (118), das eine zweite Flüssigkeitspermeabilität und eine zweite Komprimierbarkeit aufweist;
wobei mindestens eine der ersten Flüssigkeitspermeabilität größer ist als die zweite Flüssigkeitspermeabilität oder die erste Komprimierbarkeit geringer ist als die zweite Komprimierbarkeit; und
**dadurch gekennzeichnet, dass**:
das erste poröse Material und das zweite poröse Material in dem porösen Medium so angeordnet sind, dass mindestens eines von Folgendem gilt:
die proximale Zone weist eine Flüssigkeitspermeabilität auf, die größer ist als die Flüssigkeitspermeabilität der distalen Zone; oder
die proximale Zone weist eine Komprimierbarkeit auf, die geringer ist als eine Komprimierbarkeit der distalen Zone.

2. Flüssigkeitsammelvorrichtung nach Anspruch 1, wobei die erste Flüssigkeitspermeabilität größer ist als die zweite Flüssigkeitspermeabilität und die proximale Zone eine Flüssigkeitspermeabilität aufweist, die größer ist als eine Flüssigkeitspermeabilität, die von der distalen Zone aufgewiesen wird.

3. Flüssigkeitsammelvorrichtung nach Anspruch 2, wobei mindestens eines von Folgendem gilt:
das erste poröse Material weist eine Zahl an Poren pro cm auf, die größer ist als die Zahl an Poren pro cm, die von dem zweiten porösen Material aufgewiesen wird; oder
das erste poröse Material weist eine prozentuale Porosität auf, die größer ist als eine prozentuale Porosität, die von dem zweiten porösen Material aufgewiesen wird.

4. Flüssigkeitsammelvorrichtung nach einem der Ansprüche 2 oder 3, wobei mindestens eines von Folgendem gilt:
das erste poröse Material weist eine Dichte auf, die größer ist als eine Dichte, die von dem zweiten porösen Material aufgewiesen wird; oder
das erste poröse Material weist eine Hydrophilie auf, die größer ist als eine Hydrophilie, die von dem zweiten porösen Material aufgewiesen wird.

5. Flüssigkeitsammelvorrichtung nach einem der Ansprüche 2-4, wobei das poröse Medium mindestens ein intermediäres poröses Material einschließt, wobei das mindestens eine zusätzliche poröse Material eine Flüssigkeitspermeabilität aufweist, die geringer ist als die erste Flüssigkeitspermeabilität und größer ist als die zweite Flüssigkeitspermeabilität.

6. Flüssigkeitsammelvorrichtung nach einem der Ansprüche 1-5, wobei die erste Komprimierbarkeit geringer ist als die zweite Komprimierbarkeit und die proximale Zone eine Komprimierbarkeit aufweist, die geringer ist als eine Komprimierbarkeit, die von der distalen Zone aufgewiesen wird.

7. Flüssigkeitsammelvorrichtung nach Anspruch 6, wobei das erste poröse Material einen durchschnittlichen Faserdurchmesser aufweist, der größer ist als ein durchschnittlicher Faserdurchmesser, der von dem zweiten porösen Material aufgewiesen wird.

8. Flüssigkeitsammelvorrichtung nach einem der Ansprüche 6 oder 7, wobei mindestens eines von Folgendem gilt:
das erste poröse Material weist eine Zahl an Poren pro cm auf, die größer ist als die Zahl an Poren pro cm, die von dem zweiten porösen Material aufgewiesen wird;
das erste poröse Material weist einen Elastizitätsmodul auf, der größer ist als ein Elastizitätsmodul, der von dem zweiten porösen Material aufgewiesen wird;
das erste poröse Material weist eine Streckgrenze auf, die größer ist als eine Streckgrenze, die von dem zweiten porösen Material aufgewiesen wird; oder
das erste poröse Material weist eine Faserverschlingung auf, die größer ist als eine Faserverschlingung, die von dem zweiten porösen Material aufgewiesen wird.

9. Flüssigkeitsammelvorrichtung nach einem der Ansprüche 6-8, wobei das erste poröse Material und das zweite poröse Material aus isotropen Materialien gebildet sind, die unterschiedlich orientiert sind.

10. Flüssigkeitsammelvorrichtung nach einem der Ansprüche 1-9, wobei das poröse Medium eine Außenoberfläche aufweist, die durch die mindestens eine Öffnung freiliegt, wobei die Außenoberfläche durch das erste poröse Material und das zweite poröse Material gebildet ist.

11. Flüssigkeitsammelvorrichtung nach Anspruch 10, wobei sich ein Abschnitt des ersten porösen Materials hinter mindestens einem Abschnitt des zweiten porösen Materials erstreckt.

12. Flüssigkeitsammelvorrichtung nach einem der Ansprüche 10 oder 11, wobei das erste poröse Material mindestens eine Seitenoberfläche einschließt, die sich von der Außenoberfläche des porösen Materials nach innen erstreckt, wobei das zweite poröse Material die mindestens eine Seitenoberfläche des ersten Abschnittsmaterials vollständig umschließt.

13. Flüssigkeitsammelvorrichtung nach einem der Ansprüche 1-9, wobei das poröse Medium eine Außenoberfläche aufweist, die durch die mindestens eine Öffnung freiliegt, wobei die Außenoberfläche nur durch das erste poröse Material gebildet ist.

14. Flüssigkeitsammelvorrichtung nach einem der Ansprüche 1-13,
wobei eine Dicke von mindestens einem Abschnitt des ersten Abschnitts entlang einer Richtung abnimmt, die sich von dem proximalen Endbereich zu dem distalen Endbereich erstreckt.

15. Flüssigkeitssammelsystem, umfassend:
die Flüssigkeitsammelvorrichtung nach einem der Ansprüche 1-14;
einen Flüssigkeitsspeicherbehälter (1052); und
eine Vakuumquelle (1054);
wobei die Kammer der Flüssigkeitsammelvorrichtung, der Flüssigkeitsspeicherbehälter und die Vakuumquelle jeweils derart miteinander in Flüssigkeitsverbindung stehen, dass, wenn eine oder mehrere Körperflüssigkeiten in der Kammer vorhanden sind, ein von der Vakuumquelle zu der Kammer der Flüssigkeitsammelvorrichtung bereitgestelltes Vakuum die eine oder mehrere Körperflüssigkeiten aus der Kammer entfernt und die Körperflüssigkeiten in den Flüssigkeitsspeicherbehälter abführt.

## Revendications

1. Ensemble de collecte de fluide (100), comprenant :
une couche imperméable aux fluides (102) incluant une région d'extrémité proximale (104) et une région d'extrémité distale (106), la couche imperméable aux fluides délimitant au moins une ouverture (108), une chambre (110) et une sortie de fluide (112) ; et
un milieu poreux (114) disposé dans la chambre, le milieu poreux incluant :
une zone proximale (120) s'étendant à partir ou à proximité de la région d'extrémité proximale de la couche imperméable aux fluides ;
une zone distale (122) s'étendant à partir ou à proximité de la région d'extrémité distale de la couche imperméable aux fluides jusqu'à la zone proximale ;
un premier matériau poreux (116) présentant une première perméabilité aux fluides et une première compressibilité ; et
un deuxième matériau poreux (118) présentant une deuxième perméabilité aux fluides et une deuxième compressibilité ;
dans lequel la première perméabilité aux fluides est supérieure à la deuxième perméabilité aux fluides et/ou la première compressibilité est inférieure à la deuxième compressibilité ; et
**caractérisé en ce que** :
le premier matériau poreux et le deuxième matériau poreux sont disposés dans le milieu poreux de telle sorte que :
la zone proximale présente une perméabilité aux fluides qui est supérieure à la perméabilité aux fluides de la zone distale ; et/ou
la zone proximale présente une compressibilité qui est inférieure à une compressibilité de la zone distale.

2. Ensemble de collecte de fluide selon la revendication 1, dans lequel la première perméabilité aux fluides est supérieure à la deuxième perméabilité aux fluides et la zone proximale présente une perméabilité aux fluides qui est supérieure à une perméabilité aux fluides présentée par la zone distale.

3. Ensemble de collecte de fluide selon la revendication 2, dans lequel :
le premier matériau poreux présente un nombre de pores par cm qui est supérieur à un nombre de pores par cm présenté par le deuxième matériau poreux ; et/ou
le premier matériau poreux présente un pourcentage de porosité qui est supérieur à un pourcentage de porosité présenté par le deuxième matériau poreux.

4. Ensemble de collecte de fluide selon l'une quelconque des revendications 2 ou 3, dans lequel :
le premier matériau poreux présente une densité qui est supérieure à une densité présentée par le deuxième matériau poreux ; et/ou
le premier matériau poreux présente une hydrophilie qui est supérieure à une hydrophilie présentée par le deuxième matériau poreux.

5. Ensemble de collecte de fluide selon l'une quelconque des revendications 2 à 4, dans lequel le milieu poreux inclut au moins un matériau poreux intermédiaire, l'au moins un matériau poreux supplémentaire présentant une perméabilité aux fluides qui est inférieure à la première perméabilité aux fluides et supérieure à la deuxième perméabilité aux fluides.

6. Ensemble de collecte de fluide selon l'une quelconque des revendications 1 à 5, dans lequel la première compressibilité est inférieure à la deuxième compressibilité et la zone proximale présente une compressibilité qui est inférieure à une compressibilité présentée par la zone distale.

7. Ensemble de collecte de fluide selon la revendication 6, dans lequel le premier matériau poreux présente un diamètre moyen de fibres qui est supérieur à un diamètre moyen de fibres présenté par le deuxième matériau poreux.

8. Ensemble de collecte de fluide selon l'une quelconque des revendications 6 ou 7, dans lequel :
le premier matériau poreux présente un nombre de pores par cm qui est supérieur à un nombre de pores par cm présenté par le deuxième matériau poreux ; et/ou
le premier matériau poreux présente un module de Young qui est supérieur à un module de Young présenté par le deuxième matériau poreux ; et/ou
le premier matériau poreux présente une limite d'élasticité qui est supérieure à une limite d'élasticité présentée par le deuxième matériau poreux ; et/ou
le premier matériau poreux présente un enchevêtrement de fibres qui est supérieur à un enchevêtrement de fibres présenté par le deuxième matériau poreux.

9. Ensemble de collecte de fluide selon l'une quelconque des revendications 6 à 8, dans lequel le premier matériau poreux et le deuxième matériau poreux sont formés à partir de matériaux isotropes orientés différemment.

10. Ensemble de collecte de fluide selon l'une quelconque des revendications 1 à 9, dans lequel le milieu poreux présente une surface externe qui est exposée à travers l'au moins une ouverture, la surface externe étant formée par le premier matériau poreux et le deuxième matériau poreux.

11. Ensemble de collecte de fluide selon la revendication 10, dans lequel une partie du premier matériau poreux s'étend derrière au moins une partie du deuxième matériau poreux.

12. Ensemble de collecte de fluide selon l'une quelconque des revendications 10 ou 11, dans lequel le premier matériau poreux inclut au moins une surface latérale s'étendant vers l'intérieur à partir de la surface externe du matériau poreux, le deuxième matériau poreux entourant complètement l'au moins une surface latérale du premier matériau poreux.

13. Ensemble de collecte de fluide selon l'une quelconque des revendications 1 à 9, dans lequel le milieu poreux présente une surface externe qui est exposée à travers au moins une ouverture, la surface externe étant formée uniquement par le premier matériau poreux.

14. Ensemble de collecte de fluide selon l'une quelconque des revendications 1 à 13,
dans lequel une épaisseur d'au moins une partie de la première partie diminue le long d'une direction s'étendant à partir de la région d'extrémité proximale vers la région d'extrémité distale.

15. Système de collecte de fluide, comprenant :
l'ensemble de collecte de fluide selon l'une quelconque des revendications 1 à 14 ;
un récipient de stockage de fluide (1052) ; et
une source de vide (1054) ;
dans lequel la chambre de l'ensemble de collecte de fluide, le récipient de stockage de fluide et la source de vide sont en communication fluidique les uns avec les autres, de sorte que, lorsqu'un ou plusieurs fluides corporels sont présents dans la chambre, un vide fourni depuis la source de vide vers la chambre de l'ensemble de collecte de fluide retire les un ou plusieurs fluides corporels de la chambre et dépose les fluides corporels dans le récipient de stockage de fluide.
